# DEMANDE DE BREVET EUROPEEN

(11) **EP 3 369 473 A1**
(43) Date de publication de la demande: **05.09.2018**
(21) Numéro de dépôt: 18156815.5
(22) Date de dépôt: 14.02.2018
(51) Int. Cl.: B01D 53/22, C10L 3/10

(54) **INSTALLATION ET PROCEDE POUR LE TRAITEMENT PAR PERMEATION MEMBRANAIRE D'UN FLUX GAZEUX D'ALIMENTATION COMPRENANT DU METHANE ET DU DIOXYDE DE CARBONE**

(30) Priorité: 02.03.2017 FR 1751688
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: PRINCE, Guenael, 38120 SAINT EGREVE (FR); PAGET, Nicolas, 38400 Saint Martin d'Hères (FR); ZICK, Golo, 38600 FONTAINE (FR)
(74) Mandataire: Laigneau, Amandine

(57) **Abrégé**

Installation pour le traitement par perméation membranaire d'un flux gazeux d'alimentation (6) comprenant au moins du méthane et du dioxyde de carbone pour produire un flux gazeux (12) enrichi en méthane comprenant :
- une première unité (1) de séparation par membrane permettant de recevoir le flux gazeux d'alimentation et de produire un premier perméat (4) enrichi en dioxyde de carbone et un premier rétentat (7) enrichi en méthane,
- une seconde unité (2) de séparation par membrane permettant de recevoir le premier rétentat (7) et de produire un second perméat (5) enrichi en dioxyde de carbone et un second rétentat (8) enrichi en méthane,
- un éjecteur gaz-gaz (11) permettant d'augmenter la pression du premier perméat (4) à une pression comprise entre 2 et 6 bar, plus préférentiellement entre 3 et 4bar.
- une troisième unité (3) de séparation par membrane permettant de recevoir le premier perméat (4) comprimé dans l'éjecteur et de produire un troisième rétentat (9) enrichi en méthane et un troisième perméat (10) enrichi en CO2.

## Description

La présente invention est relative à une installation et à un procédé de traitement par perméation membranaire d'un courant gazeux contenant au moins du méthane et du dioxyde de carbone pour produire un courant gazeux riche en méthane.

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH4) et du dioxyde de carbone (CO2) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur à vis lubrifiée. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer in fine de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO2 est élevée; elle est donc particulièrement performante pour séparer le CO2 présent dans le biogaz, et en particulier dans le gaz de décharge.

Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH4/CO2, devant permettre la production d'un gaz contenant en fonction de son utilisation plus de 85% de CH4, de préférence plus de 95% de CO2, plus préférentiellement plus de 97,5% de CH4, tout en minimisant les pertes de CH4 dans le gaz résiduaire et le coût d'épuration, ce dernier étant pour une part importante lié à la consommation électrique du dispositif de compression du gaz en amont des membranes.

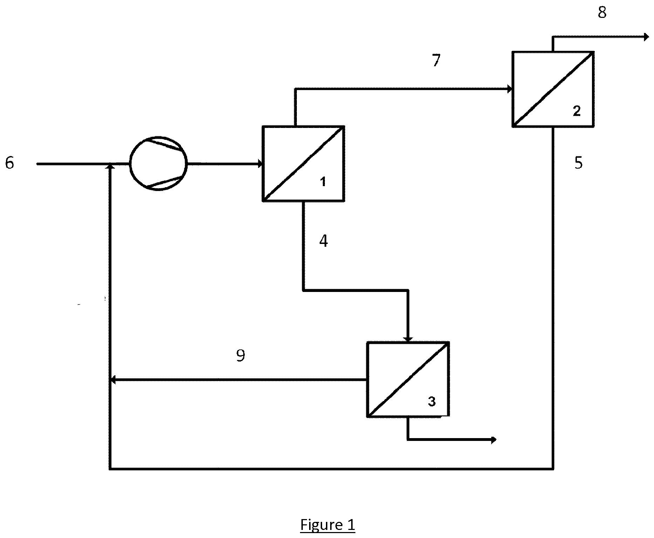

On connait la solution consistant à utiliser un système membranaire à trois étages (figure 1), dans lequel le perméat 4 du 1er étage subit une seconde séparation dans le troisième étage de membranes, avant d'être mélangé au perméat 5 du 2ème étage, pour être recyclé. Ce système à trois étages est utilisé sans recompression du perméat du 1er étage, le perméat du 2ème étage et le résiduaire du 3eme étage sont recyclés à l'entrée du système membranaire. Ce système à trois étages de membranes améliore le rendement en méthane par rapport à un système à deux étages de membranes.

Un paramètre clé de la configuration 3 étages est la pression du perméat du premier étage qui est la pression d'alimentation du troisième étage. Du coup deux objectifs contradictoires s'opposent :
1. La pression doit être minimisée afin d'augmenter la performance du premier étage ;
2. La pression doit être maximisée afin d'augmenter la performance du troisième étage ou de diminuer le nombre de modules membranaires à installer.

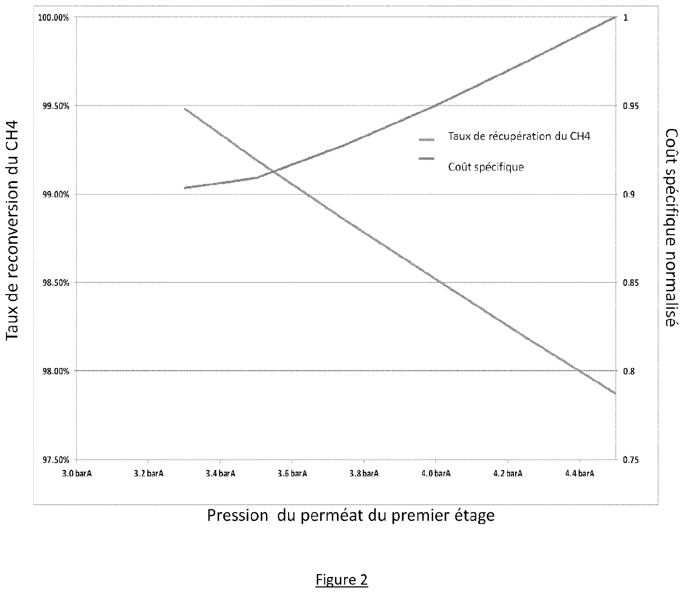

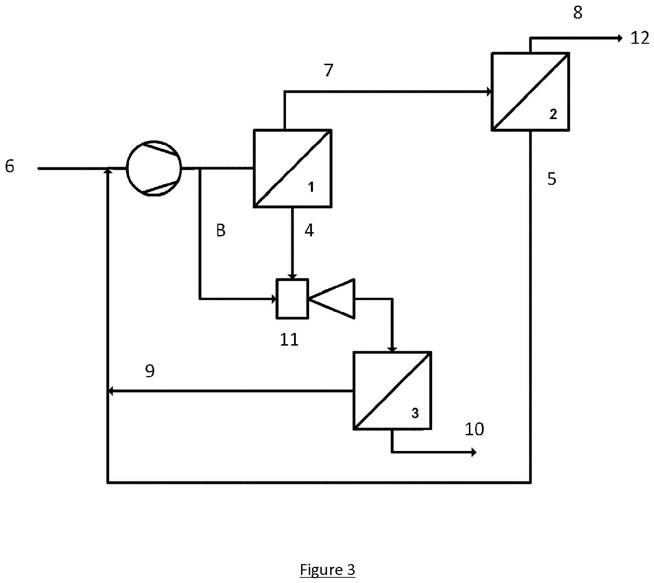

Pour démontrer l'influence de la pression du perméat du premier étage, la figure 2 montre l'évolution du rendement CH4 et du coût spécifique normalisé comme fonction de la pression du perméat du premier étage.

La figure 2 montre que la minimisation de la pression est plus importante si tous les autres paramètres sont gardés.

Afin de profiter d'une performance maximale du troisième étage il serait néanmoins souhaitable d'augmenter sa pression d'alimentation, comme il peut être réalisé à l'aide d'un compresseur mécanique.

Une solution selon l'invention est une installation pour le traitement par perméation membranaire d'un flux gazeux d'alimentation 6 comprenant au moins du méthane et du dioxyde de carbone pour produire un flux gazeux 12 enrichi en méthane comprenant :
- une première unité 1 de séparation par membrane permettant de recevoir le flux gazeux d'alimentation et de produire un premier perméat 4 enrichi en dioxyde de carbone et un premier rétentat 7 enrichi en méthane,
- une seconde unité 2 de séparation par membrane permettant de recevoir le premier rétentat 7 et de produire un second perméat 5 enrichi en dioxyde de carbone et un second rétentat 8 enrichi en méthane,
- un éjecteur gaz-gaz 11 permettant d'augmenter la pression du premier perméat 4 à une pression comprise entre 2 et 6 bar, plus préférentiellement entre 3 et 4bar.
- une troisième unité 3 de séparation par membrane permettant de recevoir le premier perméat 4 comprimé dans l'éjecteur et de produire un troisième rétentat 9 enrichi en méthane et un troisième perméat 10 enrichi en CO2.

Selon le cas, l'installation selon l'invention peut présenter une ou plusieurs des caractéristiques suivantes :
- ladite installation comprend un moyen d'acheminement d'une partie B du flux gazeux d'alimentation vers l'éjecteur gaz-gaz et l'éjecteur gaz-gaz est un éjecteur gaz-gaz employant comme gaz moteur la partie B du flux gazeux d'alimentation,
- ladite installation comprend un compresseur permettant d'augmenter la pression du flux gazeux d'alimentation à une pression supérieure à 8bar, plus préférentiellement supérieur à 13bar en amont de la première unité de séparation par membrane,
- ladite installation comprend une quatrième unité de séparation par membrane permettant de recevoir le troisième perméat et de produire un quatrième rétentat enrichi en méthane et un quatrième perméat enrichi en CO2,
- ladite installation comprend des moyens de recyclage conjoint du troisième rétentat et du second perméat en amont du compresseur,
- ladite installation comprend des moyens de recyclage conjoint du quatrième rétentat et du second perméat en amont du compresseur,
- ladite installation comprend des moyens d'évacuation du troisième perméat en dehors de l'installation,
- ladite installation comprend des moyens d'évacuation du quatrième rétentat en dehors de l'installation,
- les membranes des trois unités de séparation par membrane ont la même sélectivité ou des sélectivités différentes.

La présente invention a également pour objet un procédé de traitement par perméation membranaire d'un flux gazeux d'alimentation 6 comprenant au moins du méthane et du dioxyde de carbone pour produire un flux gazeux 12 enrichi en méthane, mettant en oeuvre une installation telle que définie dans l'invention et comprenant :
a) une première étape de séparation membranaire du flux gazeux d'alimentation dans la première unité 1 de séparation par membrane produisant un premier perméat 4 enrichi en dioxyde de carbone et un premier rétentat 7 enrichi en méthane,
b) une deuxième étape de séparation membranaire du premier rétentat 7 dans la deuxième unité de séparation 2 par membrane produisant un second perméat 5 enrichi en dioxyde de carbone et un second rétentat 8 enrichi en méthane,
c) une étape de compression du premier perméat 4 à une pression comprise entre 2 et 6bar au moyen de l'éjecteur gaz-gaz 11,
d) une troisième étape de séparation membranaire du premier perméat 4 comprimé dans l'éjecteur 11 dans la troisième unité 3 de séparation par membrane produisant un troisième rétentat 9 enrichi en méthane et un troisième perméat 10 enrichi en CO2.

Selon le cas, le procédé selon l'invention peut présenter une ou plusieurs des caractéristiques ci-dessous :
- l'éjecteur gaz-gaz 11 emploie comme gaz moteur une partie B du flux gazeux d'alimentation.
- en amont de la première unité 1 de séparation membranaire le flux gazeux d'alimentation 6 est comprimé à une pression supérieure à 8 bar, plus préférentiellement supérieur à 13 bar.
- ledit procédé comprend une quatrième étape de séparation membranaire du troisième perméat produisant un quatrième rétentat enrichi en méthane et un quatrième perméat enrichi en CO2.
- le troisième rétentat 9 et le second perméat 5 sont recyclés conjointement en amont du compresseur.
- le quatrième rétentat et le second perméat sont recyclés conjointement en amont du compresseur.

Dans le cadre de l'invention, le flux gazeux d'alimentation est de préférence du biogaz provenant par exemple d'un digesteur, d'un fermenteur, d'une déchèterie ou STEP (STEP = STation d'EPuration)

L'installation et le procédé selon l'invention en augmentant la pression du premier perméat à une pression comprise entre 2 et 6 bar, c'est-à-dire en effectuant une augmentation « légère » de pression, permettent soit de diminuer la surface membranaire à installer au niveau du troisième étage et du coup de diminuer les coûts d'investissement tout en gardant le rendement constant, soit d'augmenter la performance de l'installation / du procédé selon l'invention.

L'éjecteur gaz-gaz employant comme gaz moteur le flux gazeux d'alimentation du premier étage, il n'y a aucun risque de pollution. L'éjecteur possède en plus l'avantage de ne comporter aucune pièce mobile.

## Revendications

1. Installation pour le traitement par perméation membranaire d'un flux gazeux d'alimentation (6) comprenant au moins du méthane et du dioxyde de carbone pour produire un flux gazeux (12) enrichi en méthane comprenant :
- une première unité (1) de séparation par membrane permettant de recevoir le flux gazeux d'alimentation et de produire un premier perméat (4) enrichi en dioxyde de carbone et un premier rétentat (7) enrichi en méthane,
- une seconde unité (2) de séparation par membrane permettant de recevoir le premier rétentat (7) et de produire un second perméat (5) enrichi en dioxyde de carbone et un second rétentat (8) enrichi en méthane,
- un éjecteur gaz-gaz (11) permettant d'augmenter la pression du premier perméat (4) à une pression comprise entre 2 et 6 bar, plus préférentiellement entre 3 et 4bar.
- une troisième unité (3) de séparation par membrane permettant de recevoir le premier perméat (4) comprimé dans l'éjecteur et de produire un troisième rétentat (9) enrichi en méthane et un troisième perméat (10) enrichi en CO2.

2. Installation selon la revendication 1, **caractérisée en ce que** ladite installation comprend un moyen d'acheminement d'une partie B du flux gazeux d'alimentation de la première unité de séparation par membrane vers l'éjecteur gaz-gaz et l'éjecteur gaz-gaz est un éjecteur employant comme gaz moteur la partie B du flux gazeux d'alimentation.

3. Installation selon l'une des revendications 1 ou 2, **caractérisée en ce que** ladite installation comprend un compresseur permettant d'augmenter la pression du flux gazeux d'alimentation à une pression supérieure à 8bar, plus préférentiellement supérieur à 13bar en amont de la première unité de séparation par membrane.

4. Installation selon la revendication 3, **caractérisée en ce que** ladite installation comprend une quatrième unité de séparation par membrane permettant de recevoir le troisième perméat et de produire un quatrième rétentat enrichi en méthane et un quatrième perméat enrichi en CO2.

5. Installation selon la revendication 3, **caractérisée en ce que** ladite installation comprend des moyens de recyclage conjoint du troisième rétentat et du second perméat en amont du compresseur.

6. Installation selon la revendication 4, **caractérisée en ce que** ladite installation comprend des moyens de recyclage conjoint du quatrième rétentat et du second perméat en amont du compresseur.

7. Installation selon la revendication 3, **caractérisée en ce que** la dite installation comprend des moyens d'évacuation du troisième perméat en dehors de l'installation.

8. Installation selon la revendication 4, **caractérisée en ce que** ladite installation comprend des moyens d'évacuation du quatrième rétentat en dehors de l'installation.

9. Installation selon l'une des revendications 1 à 8, **caractérisée en ce que** les membranes des trois unités de séparation par membrane ont la même sélectivité ou des sélectivités différentes.

10. Procédé de traitement par perméation membranaire d'un flux gazeux d'alimentation (6) comprenant au moins du méthane et du dioxyde de carbone pour produire un flux gazeux (12) enrichi en méthane, mettant en oeuvre une installation telle que définie dans l'une des revendications 1 à 9 et comprenant :
a) une première étape de séparation membranaire du flux gazeux d'alimentation dans la première unité (1) de séparation par membrane produisant un premier perméat (4) enrichi en dioxyde de carbone et un premier rétentat (7) enrichi en méthane,
b) une deuxième étape de séparation membranaire du premier rétentat (7) dans la deuxième unité de séparation (2) par membrane produisant un second perméat (5) enrichi en dioxyde de carbone et un second rétentat (8) enrichi en méthane,
c) une étape de compression du premier perméat (4) à une pression comprise entre 2 et 6bar au moyen de l'éjecteur gaz-gaz (11),
d) une troisième étape de séparation membranaire du premier perméat (4) comprimé dans l'éjecteur (11) dans la troisième unité (3) de séparation par membrane produisant un troisième rétentat (9) enrichi en méthane et un troisième perméat (10) enrichi en CO2.

11. Procédé selon la revendication 10, **caractérisé en ce que** l'éjecteur gaz-gaz (11) emploie comme gaz moteur une partie B du flux gazeux d'alimentation.

12. Procédé selon l'une des revendications 10 ou 11, **caractérisé en ce qu'**en amont de la première unité de séparation membranaire le flux gazeux (6) d'alimentation est comprimé à une pression supérieure à 8bar, plus préférentiellement supérieure à 13bar.

13. Procédé selon la revendication 12, **caractérisé en ce que** ledit procédé comprend une quatrième étape de séparation membranaire du troisième perméat produisant un quatrième rétentat enrichi en méthane et un quatrième perméat enrichi en CO2.

14. Procédé selon la revendication 12, **caractérisé en ce que** le troisième rétentat (9) et le second perméat (5) sont recyclés conjointement en amont du compresseur.

15. Procédé selon la revendication 13, **caractérisé en ce que** le quatrième rétentat et le second perméat sont recyclés conjointement en amont du compresseur.
